# EUROPEAN PATENT APPLICATION

(11) **EP 2 821 106 A1**
(43) Date of publication of application: **07.01.2015**
(21) Application number: 13175264.4
(22) Date of filing: 05.07.2013
(51) Int. Cl.: A61P 35/00, A61K 35/74, A61K 36/38, A61K 36/71, A61K 36/88

(54) **Composition and use thereof for the treatment of tumor indications**

(71) Applicant: Cellquantum GmbH, 82024 Taufkirchen (DE)
(72) Inventor: Temper, Rupert, 4342 Baumgartenberg (AT)
(74) Representative: Vos, Derk

(57) **Abstract**

The present disclosure relates to a composition comprising several plants, preferably extracts, useful in the treatment and prevention of benign and malign tumor indications, the use of such composition for the treatment or prevention of benign and malign tumor indications, and a method for the production of such composition. The composition comprises the compounds *carica papaya* and *pineapple,* and at least one compound selected from the group consisting of *agaricus subrufescens; cyanobacteria; sambucus;* and *hypericum perforatum.*

## Description

### BACKGROUND OF THE INVENTION

### Field of the invention

The present disclosure relates to a composition comprising several plants, preferably extracts, useful in the treatment and prevention of benign and malign tumor indications, the use of such composition for the treatment or prevention of benign and malign tumor indications, and a method for the production of such composition.

### Background of the invention

Cancer, such as benign and malign tumor indications, also referred to as benign and malign tumors, is still one of the most threatening diseases. This is also partly due to the difficulty modem medicine still has in curing cancer.

Many different strategies to cure cancer are known. However, the newly developed drugs are usually limited in their applicability and often have severe side effects.

There is thus still a need for a drug that is useful in the treatment or prevention of cancer, especially benign and malign tumor indications, without severe side effects.

### SUMMARY OF THE INVENTION

In a first aspect, the present disclosure provides a composition as disclosed in claim 1.

In another aspect, the present disclosure provides the use of a composition of the present disclosure in the treatment or prevention of benign and malign tumor indications, preferably tumors to the brain, colorectal cancer with or without metastases, prostatic cancer with or without metastases, and benign tumor indications.

In another aspect, the present disclosure provides a method for the treatment or prevention of a benign and malign tumor indication in an animal, comprising administering to an animal in need thereof, an effective amount of a composition of the present disclosure.

In another aspect, the present disclosure provides the use of a composition of the present disclosure for the preparation of a medicament, preferably for the treatment or prevention of a benign and/or malign tumor indication.

In still another aspect, the present disclosure provides a method for the preparation of a composition of the present disclosure.

### DETAILED DESCRIPTION OF THE INVENTION

The compositions of the first aspect of the present invention comprise the compounds *carica papaya* and *pineapple,* and at least one compound selected from the group consisting of *agaricus subrufescens; cyanobacteria; sambucus;* and *hypericum perforatum.*

In a preferred embodiment, the composition comprises the compounds *carica papaya* and *pineapple,* and at least one compound selected from the group consisting of *agaricus subrufescens* and *cyanobacteria.*

In another preferred embodiment, the composition comprises the compounds *carica papaya; pineapple; agaricus subrufescens;* and *cyanobacteria.*

In another preferred embodiment, the composition additionally comprises at least one compound selected from the group consisting of *sambucus* and *hypericum perforatum.*

In another preferred embodiment, the *cyanobacteria* is *spirulina.*

In another preferred embodiment, the *sambucus* is *sambucus flos.*

In another preferred embodiment, the composition comprises the compounds *carica papaya,* preferably green *carica papaya; pineapple; agaricus subrufescens;* and *cyanobacteria,* preferably *spirulina;* and at least one compound selected from the group consisting of *sambucus,* preferably *sambucus flos;* and *hypericum perforatum.*

In another preferred embodiment, the composition comprises the compounds *carica papaya,* preferably green *carica papaya; pineapple; agaricus subrufescens; cyanobacteria,* preferably *spirulina; sambucus,* preferably *sambucus flos;* and *hypericum perforatum.*

In another preferred embodiment, the compound that is contained in the composition is a plant or algae, preferably the compound is an extract of said plant or algae, respectively, and more preferably an aqueous extract.

In another preferred embodiment, any of the compounds selected from the group consisting of *carica papaya,* preferably green *carica papaya; pineapple; agaricus subrufescens; cyanobacteria,* preferably *spirulina; sambucus,* preferably *sambucus flos;* and *hypericum perforatum,* is present as an extract, preferably an aqueous extract, in an amount of from 50 to 300 ml, preferably from 70 to 260 ml, based on one liter of composition.

In another preferred embodiment, any of the compounds selected from the group consisting of *carica papaya,* preferably green *carica papaya; pineapple; cyanobacteria,* preferably *spirulina; sambucus,* preferably *sambucus flos;* and *hypericum perforatum,* is present as an extract, preferably an aqueous extract.

In another preferred embodiment, the composition further comprises a sugar, preferably sucrose.

In another preferred embodiment, the composition further comprises oxygen (O₂), preferably containing oxygen (O₂) in an amount of more than 23 mg/l.

In another preferred embodiment, the composition further comprises calcium phosphate, potassium citrate, silicon dioxide, sodium chloride, magnesium citrate, lithium carbonate, zinc gluconate, hypericum perforatum, and cyanobacteria or agaricus subrufescens.

In another preferred embodiment, the composition further comprises colloidal silver.

In another preferred embodiment, the composition further comprises vitamins and/or minerals.

In another preferred embodiment, the composition is an aqueous composition.

The compositions of the present disclosure may be used for the treatment or prevention of a benign and malign tumor indication. In other words, the compositions of the present disclosure are useful in the treatment or prevention of a benign and malign tumor indication.

In a preferred embodiment, the benign and malign tumor indication is selected from the group consisting of
K1. Tumors to the brain;
K2. Colorectal cancer with or without metastases;
K3. Prostatic cancer with or without metastases; and
K4. Benign tumor indications.

In another preferred embodiment, the composition is administered orally, intravenously, subcutaneously, or topically, preferably orally or intravenously, and more preferably orally, when used in the treatment or prevention of a benign and malign tumor indication.

In another preferred embodiment, the composition is administered once daily up to 10 times daily, preferably once daily up to five times daily, and more preferably three times daily.

In still another preferred embodiment, the composition treatment is applied for at least four weeks, preferably at least 12 weeks, and more preferably between 12 weeks and 48 weeks, when the composition is used in the treatment or prevention of a benign and malign tumor indication.

In another preferred embodiment, the composition is co-administered together with at least one additive, which is not necessarily taken at the same time and/or mixed with the composition.

In a further preferred embodiment, the at least one additive is selected from the group consisting of
(a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof;
(b) colloidal silver;
(c) oxygen water, preferably containing oxygen (O₂) in an amount of more than 23 mg/l;
(d) vitamin(s); and
(e) mineral(s).

In another further preferred embodiment, the composition is co-administered together with at least the additive
(a) composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof.

In another further preferred embodiment, the composition is co-administered together with at least the additive
(a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof; and
(b) colloidal silver.

In another further preferred embodiment, the composition is co-administered together with at least the additives
(a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof; and
(c) oxygen water, preferably containing oxygen (O₂) in an amount of more than 23 mg/l.

In another further preferred embodiment, the composition is co-administered together with at least the additives
(a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof;
(b) colloidal silver; and
(c) oxygen water, preferably containing oxygen (O₂) in an amount of more than 23 mg/l.

In another preferred embodiment, the composition Q4 comprises a mixture of calcium phosphate, potassium citrate, silicon dioxide, sodium chloride, magnesium citrate, lithium carbonate, zinc gluconate, hypericum perforatum, and cyanobacteria or agaricus subrufescens.

The present disclosure is also directed to a composition as disclosed in the present disclosure for use in the treatment or prevention of a benign and malign tumor indication. The preferred benign and malign tumor indications are listed herein.

The present disclosure is also directed to a method for the treatment or prevention of a benign and malign tumor indication in an animal, comprising administering to an animal in need thereof, an effective amount of a composition as disclosed herein. The preferred benign and malign tumor indications are listed herein.

The present disclosure is also directed to the use of a composition as disclosed in the present disclosure for the preparation of a medicament. In a preferred embodiment, the medicament is a medicament for the treatment or prevention of a benign and malign tumor indication. The preferred benign and malign tumor indications are listed herein.

Furthermore, the present disclosure is also directed to a method for the preparation of a composition as disclosed herein, wherein the composition is an aqueous composition, comprising the steps of
(a) Providing a ready to use aqueous extract of *carica papaya* and *pineapple,* and admixing at least an aqueous extract of a compound selected from the group consisting of *agaricus subrufescens; cyanobacteria; sambucus;* and *hypericum perforatum;*
(b) adding sucrose to said mixture; and
(c) optionally enriching said mixture with oxygen.

Finally, the present disclosure is also directed to a kit of part comprising
- a composition as disclosed in the present disclosure; and
- an additive comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and a combination thereof; and/or
- oxygen water, preferably containing oxygen (O₂) in an amount of more than 23 mg/l; and/or
- colloidal silver; and/or
- vitamin(s); and/or
- mineral(s); and
- optionally instructions for a dosage regimen.

It is understood that any of the preferred embodiments or compositions may be combined with each other resulting in further preferred embodiments of the present invention.

The compositions of the present disclosure are useful in the treatment of some specific types of malign and benign tumor indications. Some of the more specific types of benign and malign tumor indication are illustrated in the following in more detail.

### Tumors to the brain

Various types of cancer in the brain may be treated or prevented by the compositions of the present disclosure. Exemplary types of cancer to the brain include glioblastoma with or without metastases, anaplastic astrocytoma, and meningioma.

*Glioblastoma multiforme* (GBM) is the most common and most aggressive malignant primary brain tumor in humans, involving glial cells and accounting for 52% of all functional tissue brain tumor cases and 20% of all intracranial tumors. Despite being the most prevalent form of primary brain tumor, GBM incidence is only 2-3 cases per 100,000 people in Europe and North America. Conventional treatment can involve chemotherapy, radiation, radiosurgery, corticosteroids, antiangiogenic therapy, surgery and experimental approaches such as gene transfer.

With the exception of the brainstem gliomas, glioblastoma has the worst prognosis of any central nervous system (CNS) malignancy, despite multimodality treatment consisting of open craniotomy with surgical resection of as much of the tumor as possible, followed by concurrent or sequential chemoradiotherapy, antiangiogenic therapy with bevacizumab, gamma knife radiosurgery, and symptomatic management with corticosteroids.

*Anaplastic astrocytoma* is a WHO grade 3 type of astrocytoma. The growth rate and mean interval between onset of symptoms and diagnosis is approximately 1.5-2 years but is highly variable, being intermediate between that of low-grade astrocytomas and glioblastomas. Seizures are less common among patients with anaplastic astrocytomas compared to low-grade lesions.

*Meningiomas* are a diverse set of tumors arising from the meninges, the membranous layers surrounding the central nervous system. According to the American Brain Tumor Association, meningiomas are the most common primary brain tumor, representing one-third of all such tumors. These tumors are usually benign in nature; however, a small percentage is malignant. Symptomatic meningiomas are typically treated with either radiosurgery or conventional surgery.

### Colorectal cancer

The compositions of the present disclosure are also useful in the treatment or prevention of colorectal cancer, with or without metastases.

*Colorectal cancer,* commonly known as colon cancer or bowel cancer, is a cancer from uncontrolled cell growth in the colon or rectum (parts of the large intestine), or in the appendix. Cancers that are confined within the wall of the colon are often curable with surgery while cancer that has spread widely around the body is usually not curable and management then focuses on extending the person's life via chemotherapy and improving quality of life. Colorectal cancer is the third most commonly diagnosed cancer in the world, but it is more common in developed countries.

### Prostate cancer

The compositions of the present disclosure are also useful in the treatment or prevention of prostate cancer, with or without metastases.

Prostate cancer is a form of cancer that develops in the prostate, a gland in the male reproductive system. Most prostate cancers are slow growing; however, there are cases of aggressive prostate cancers. The cancer cells may metastasize (spread) from the prostate to other parts of the body, particularly the bones and lymph nodes. Conventionally, curative treatment generally involves surgery, various forms of radiation therapy, or, less commonly, cryosurgery; hormonal therapy and chemotherapy are generally reserved for cases of advanced disease (although hormonal therapy may be given with radiation in some cases).

### Benign Tumor Indications

A benign tumor is a mass of cells (tumor) that lacks the ability to invade neighboring tissue or metastasize. These characteristics are required for a tumor to be defined as cancerous and therefore benign tumors are non-cancerous. Also, benign tumors generally have a slower growth rate than malignant tumors and the tumor cells are usually more differentiated (cells have normal features). Benign tumors are typically surrounded by an outer surface (fibrous sheath of connective tissue) or remain with the epithelium. Common examples of benign tumors include melanocytic nevus (nevi) and uterine fibroids (leiomyomas).

### Uterine fibroids

Uterine fibroids (also referred to as myoma, leiomyoma, leiomyomata, and fibromyoma) are benign (non-cancerous) tumors that grow within the muscle tissue of the uterus. Between 20-50% of women of childbearing age have uterine fibroids. While many women do not experience any problems, symptoms can be severe enough to require treatment. Myomas may grow as a single nodule or in clusters and may range in size from 1 mm to more than 20 cm in diameter. Myomas are the most frequently diagnosed tumor of the female pelvis and the most common reason for a woman to have a hysterectomy. The cause of myomas has not actually been determined. The current treatment of uterine fibroids generally involves surgery.

### Description of ingredients of the composition

In the composition according to the present disclosure, several active agents contained in natural plants and algae are used. The term "compound" as used in the present disclosure refers to a natural plant or algae as used herein, to a part of said plant or algae, to an active agent present in said plant or algae, or to a specific single compound present in said plant or algae, or also to an extract of said plant or algae. In the following, the corresponding species are illustrated in more detail.

For the purpose of the present disclosure, if "approximately equal amounts" of the compounds are used, the difference in the amounts (by volume or mass, respectively) is less than 15 %, preferably less than 10 %, more preferably less than 5 % and most preferably less than 1 % (by volume or mass, respectively).

### Cyanobacteria

Cyanobacteria, also known as blue-green bacteria, blue-green algae, or Cyanophyta, is a phylum of bacteria that obtain their energy through photosynthesis. In the present disclosure, Arthrospira or Spirulina is preferably used as cyanobacteria. Preferably, Arthrospira platensis and Arthrospira maxima are used in the disclosure. Arthrospira is a genus of free-floating filamentous cyanobacteria characterized by cylindrical, multicellular trichomes in an open left-hand helix. Spirulina is mainly commercially produced at the Hawaii Islands and in California. The FDA (US Food and Drug Authority) rewarded under sections 201(s) and 409 of the Federal Food, Drug, and Cosmetics Act the Spirulina produced by Cyanotech Corporation of Hawaii and Earthrise Nutritionals LLC of California GRAS (Generally Recognized As Safe). Therefore these two producers would be preferred for the use in compositions of the present disclosure. Studies have shown that polysaccharide extracts increase macrophage function, antibody production and infection fighting T-cells. Polysaccharides and phycocyanin from Spirulina have shown to increase immunity in mice by enhancing bone marrow reproduction, growth of thymus and spleen and biosynthesis of serum protein.

In a preferred embodiment of the present disclosure, the dried cyanobacteria have at least 80 mg of lithium per gram of dry cyanobacteria, preferably Spirulina. *Cyanobacteria* are referred to in the present disclosure also as "algae", falling under said general term.

### Agaricus subrufescens

Agaricus subrufescens, also termed as Agaricus blazei or Agaricus blazei murill, Agaricus brasiliensis, or Agaricus rufotegulis, is a species of mushroom. Agaricus subrufescens was traditionally used to treat many common diseases like atherosclerosis, hepatitis, hyperlipidemia, diabetes, dermatitis and cancer. In vitro and in vivo, Agaricus subrufescens has shown immunomodulatory and antimutagenic properties. For the present disclosure, an aqueous extract of Agaricus subrufescens is preferably used, more preferably from the cap of Agaricus subrufescens, further preferred in the presence of sucrose.

### Carica papaya

The papaya is the fruit of the plant *Carica papaya,* the sole species in the genus Carica of the plant family Caricaceae. Two kinds of papayas are commonly grown. One has sweet, red (or orangish) flesh, and the other has yellow flesh. Either kind picked green, i.e., unripe, is called a "green" papaya. For the compositions used in the present disclosure, preferably the fruit of the green, i.e. unripe, papaya is used. In another preferred embodiment of the present disclosure, the *carica papaya* is used as an extract, preferably an aqueous extract, more preferably as an extract or aqueous extract of the green papaya.

### Pineapple

The *pineapple* (Ananas comosus) is a tropical plant in the Bromeliaceae family. Pineapple contains four distinct cysteine proteinases. The major proteinase present in extracts of plant stem is stem bromelain, whilst fruit bromelain is the major proteinase in the fruit. Two additional cysteine proteinases are present only in the stem: these are ananain and comosain. Stem bromelain, fruit bromelain and ananain are immunologically distinct. Bromelain is the most important enzyme.

The fruit and/or stem of *pineapple* may be used in the compositions of the present disclosure. For the compositions of the present disclosure, preferably an extract of the fruit and/or stem, further preferably extracts of both fruit and stem, are used. In a further preferred embodiment, the extract is an aqueous extract.

### Hypericum perforatum

St John's wort is the plant species *Hypericum perforatum,* and is also known as Tipton's weed, chase-devil, or Klamath weed. Hypericum perforatum is a perennial plant with extensive, creeping rhizomes. Its stems are erect, branched in the upper section, and can grow to 1 m high. For the compositions of the present disclosure, the whole plant of Hypericum perforatum, further preferably locally grown Hypericum perforatum, may be used. In a preferred embodiment of the present disclosure, the *hypericum perforatum* is used as an extract, preferably an aqueous extract.

### Sambucus

Sambucus, also referred to as elder or elderberry, is a genus of between 5 and 30 species of shrubs or small trees in the moschatel family, Adoxaceae. A specific genus is not preferred in the present disclosure. For use in the compositions of the present disclosure, the flowers (flos) of *sambucus* are preferred. For the purpose of the preparation of a liquid extract from sambucus, preferably the flowers (flos) is used, however, any part of the plant may be used to prepare an extract. In a further preferred embodiment, the extract is an aqueous extract.

### Preferred combinations

Preferred combinations of the above given compounds resulting in an inventive composition according to the present disclosure are summarized in Table 1 below:

**Table 1: Sub-combination of preferred compositions**

| Sub-combination No. | Agaricus Subrufescens | Cyanobacteria | Sambucus | Carica Papaya | Pineapple | Hypericum perforatum | No of ingredients |
|---|---|---|---|---|---|---|---|
| 1 | | | | X | X | X | 3 |
| 2 | | | X | X | X | | 3 |
| 3 | X | | | X | X | | 3 |
| 4 | | X | | X | X | | 3 |
| 5 | X | | X | X | X | | 4 |
| 6 | | X | X | X | X | | 4 |
| 7 | X | | | X | X | X | 4 |
| 8 | | X | | X | X | X | 4 |
| 9 | X | X | | X | X | X | 5 |
| 10 | X | X | X | X | X | | 5 |

As can be gathered also from the above given Table 1, the compositions of the present disclosure comprise at least the compounds *carica papaya* and *pineapple,* and at least one compound selected from the group consisting of *agaricus subrufescens, cyanobacteria, sambucus* and *hypericum perforatum.*

### Extracts of the plants and algae

According to the present disclosure, the composition comprising plants and algae is preferably prepared from extracts of the plants and algae. Especially preferred are aqueous extracts. It is understood that the concentration of active ingredients of the extracts of natural products, such as plants and algae, highly depend on the source of the natural products and the amount of extraction medium. The preferred extraction medium according to the present disclosure is distilled water.

A summary of possible concentrations of aqueous extracts is given in Table 2 below, based on the amount of extracted plant or algae (dried or fresh, as indicated). The plants or algae are extracted using water, preferably distilled water, at a temperature in the range of 20 to 60 °C, preferably 25 to 50 °C. The water is poured over the plant or algae in dried or fresh (i.e., non-dried) form, then allowed to brew or extract for an appropriate time, such as 1 minute to 72 hour, preferably 5 minutes to 30 minutes and in case of carica papaya 72 hours. Finally, the extracted plants or algae are filtered off to result in a filtrate containing extracts of the plant or algae.

**Table 2: Amounts of plants and algae used for preparing aqueous extraction**

| Compound | Amount used for extraction (g/100 ml distilled water) | Compound condition |
|---|---|---|
| Cyanobacteria | 1 to 40, preferably 10 to 40 | Dried |
| Agaricus Subrufescens | 1 to 50, preferably 10 to 50 | Dried |
| Pineapple | 10 to 180, preferably 100 to 180 | Fresh |
| Carica papaya | 10 to 180, preferably 100 to 180 | Fresh |
| Hypericum perforatum | 20 to 60, preferably 40 to 60 | Fresh |
| Sambucus flos | 20 to 60, preferably 40 to 60 | Fresh |

Alternatively, commercially available extracts of the corresponding plants and algae may be used in accordance with the present disclosure.

In a preferred embodiment, the plants and algae of the present disclosure are extracted with aqueous extraction media, preferably water, more preferably distilled water, resulting in an aqueous extract of the plant or algae. However, also other extraction media may be used. Examples of other extraction media include, but are not limited to organic solvents, such as alcohols, preferably methanol, ethanol and propanol, acetic acid, acetic acid esters, acetyl acetone, and mixtures thereof, as well as mixtures with water.

The compositions of the present disclosure may then initiate an immune modulation, leading to the destruction and phagocytosis of the tumor cells in addition to an antiinflammatory action.

### Kits

The disclosure further provides kits that can simplify the handling and administration of a composition of the present disclosure to an animal.

In one embodiment, a kit of the disclosure comprises a unit dosage form of a composition of the present disclosure. In one embodiment, the unit dosage form comprises a first container, which can be sterile, containing an effective amount of a composition of the present disclosure and a pharmaceutically acceptable carrier or excipient. The kit can further comprise a label or printed instructions instructing the use of the composition of the present disclosure to treat or prevent cancer. The kit can further comprise a unit dosage form of a second therapeutic agent, for example, a second container containing an effective amount of the second therapeutic agent and a pharmaceutically acceptable carrier or excipient. In another embodiment, the kit comprises a container containing an effective amount of a composition of the present disclosure, an effective amount of a second therapeutic agent and a pharmaceutically acceptable vehicle, carrier, or excipient. Examples of second therapeutic agents include, but are not limited to, the below Additives 1 to 4.

Kits of the disclosure can further comprise a device that is useful for administering the unit dosage forms. Examples of such a device include, but are not limited to, a syringe, a drip bag, a patch, an inhaler, and an enema bag. The kit should not contain further chemical drugs, which could interact with the composition and therefore weaken the effectiveness of it.

The kit may additionally contain one or more of the following additives:
- Additive 1: Q4 composition
- Additive 2: Liquid colloidal silver
- Additive 3: Oxygen water
- Additive 4: Vitamins and minerals

These additives may be administered in addition to the composition of the present disclosure, but not necessarily at the same time or even mixed with it.

### Additive 1: Special Composition Q4

Additive 1 is a special composition containing compounds, such as macrominerals and trace elements, which may be beneficial to any patient suffering from a tumor. As such, Additive 1 may be helpful in treating any disease, including benign and malign tumor indications. However, as can also be gather from the list of ingredients as given in Table 3 below, Additive 1 does not contain any "drug" or "active agent" and is thus only considered as a helpful supportive dietary, however, not essential for the treatment with the compositions of the present disclosure.

**Table 3: Ingredients of Additive 1**

| No. | Ingredient | Amount [mg/liter] |
|---|---|---|
| 1 | Calcium Phosphate | 26 |
| 2 | Potassium Citrate | 38 |
| 3 | Silicon Dioxide | 9,7 |
| 4 | Sodium Chloride | 22,3 |
| 5 | Magnesium Citrate | 42 |
| 6 | Lithium Carbonate | 370 |
| 7 | Zinc Gluconate | 0,35 |
| 8 | Hypericum perforatum | 32-77 |
| 9 | Cyanobacteria or Agaricus subrufescens | 43-108 |

### Additive 2: Colloidal silver

The silver ion (Ag⁺) is bioactive and in sufficient concentration readily kills bacteria *in vitro.* Silver also kills bacteria in external wounds in living tissue, so physicians use wound dressings containing silver sulfadiazine (Ag-SD) or silver nanomaterials to treat external infections. Wound dressings containing silver are increasing in importance due to the recent increase of antibiotic-resistant bacteria, such as methicillin-resistant Staphylococcus aureus (MRSA). The disinfectant properties of silver are used in medical applications, such as urinary catheters and endotracheal breathing tubes, where the silver content is effective in reducing incidences of catheter-related urinary tract infections and ventilator-associated pneumonia (VAP), respectively. Silver is also used in bone prostheses, reconstructive orthopedic surgery and cardiac devices, as well as on surfaces and fabrics to reduce the spread of infection. The mild silver in colloidal form is used as solution.

### Additive 3: Oxygen water

Normal tab water filtered (filter type active coal filter, e.g. Carbonit Premium, with a filter pore size of 0,450 µm) enriched with oxygen with a content greater than 25 mg/l water, may be used in addition to the compositions of the present disclosure. Such oxygen enriched water is termed "oxygen water" for the purpose of the present disclosure.

For the present disclosure, if water is enriched with oxygen, the oxygen may be dissolved in water, such as physically or chemically dissolved in the water, or adhere to any of the compounds of the present disclosure.

### Additive 4: Vitamins and minerals

The treatment may also be accompanied by the application of natural vitamins and/or minerals. There is no specific kind or source of vitamin and/or mineral, but natural sources of vitamins and minerals are preferred for their usually high bioavailability. Any combination of vitamin(s) and/or mineral(s) may be used, preferably vitamin(s) and/or mineral(s) having a high bioavailability. Exemplary vitamins are C, A, D, B12 and the like, and exemplary minerals are zinc, magnesium, calcium, potassium, and the like. Dosage of any of these vitamins and minerals is preferably 100% of the recommended daily allowance (RDA).

### Application

The compositions or the present disclosure may be applied, among others, orally, intravenously and subcutaneously. Further, the composition can also be applied topically or inhaled using a vaporizer. In a preferred embodiment of the present disclosure, the compositions are applied orally or topically. Both the oral and topical application may be used to cancer systemically or locally. In other words, also a topical application may be used to treat any cancer since the active agents of the composition are resorbed via the skin and transported via the blood or lymph to the tumor. On the other hand, the compositions of the present disclosure may also be used as a local treatment for cancers close to the site of topical application if, e.g., applied topically. In a more preferred embodiment of the present disclosure, the compositions are applied orally.

Together with the compositions of the present disclosure, also additives may be applied. These additives may be co-administered with the compositions, i.e., taken together simultaneously or even admixed with the compositions. This applies to all modes of application, especially the oral and topical application. However, the additives may also be administered separately, i.e., physically separate from the compositions, and also with a time difference. If a topical application of the composition is used, a further topical application of additives, such as Additive 1, is not necessary, however, such additive may be applied in any way described above, such as topically or orally, or both.

### Dosage

The compositions of the present disclosure are preferably applied together with Additives 1 and 2. A preferred way of application is the oral application. An exemplary dosage regime for oral application of the composition and Additives 1 to 3 for an adult patient is listed in Table 4. The dosage amount may depend on the type of tumor and its severity.

**Table 4: dosage regime**

| Substance | Amount [ml] per dose | Frequency | Time of Day |
|---|---|---|---|
| Composition of the present disclosure | 1 to 5 | 3 times daily | Morning: fasting, 30 minutes before breakfast |
| | | | Noon: 30 minutes before lunch |
| | | | Afternoon: preferably not later than 17:00, before dinner |
| Additive 1 | 3 to 10 | 3 times daily | Morning: fasting, 30 minutes before breakfast |
| | | | Noon: 30 minutes before lunch |
| | | | Afternoon not later than 17:00, before dinner |
| Additive 2 | 10 to 30 | 3 times daily | Morning: fasting, 30 minutes before breakfast |
| | | | Noon: 30 minutes before lunch |
| | | | Afternoon not later than 17:00, before dinner |
| Additive 3 | 500 to 1500 | evenly distributed | Small amounts evenly distributed during the day |

### BRIEF DESCRIPTION OF THE DRAWINGS

Exemplary embodiments of the invention will be described in the following with reference to the following drawings:
Fig. 1 showing the complete remission of a rectum adeno-carcinoma in Case Report 1;
Fig. 2 showing the comparison of median overall survival of a patient with recurrent glioblastoma in Case Report 2;
Fig. 3 showing a complete remission of an uterine myoma in Case Report 4;
Fig. 4 showing the complete remission of an uterine myoma as given in Case Report 6;
Fig. 5 showing a complete remission of a meningeoma as given in Case Report 7.

### EXPERIMENTAL SECTION

In the following, the present invention is illustrated in more detail. However, it is understood that the scope of protection is only determined by the attached claims, not being restricted to any of the following Examples. The following Examples are set forth to assist in understanding the invention and should not be construed as specifically limiting the invention described and claimed herein. Such variations of the invention, including the substitution of all equivalents now known or later developed, that would be within the purview of those skilled in the art, and changes in formulation or changes in experimental design, are to be considered to fall within the scope of the invention incorporated herein.

### Preparation of composition

### Production process of Exemplary Composition A with ready to use extracts

Exemplary Composition A of the present disclosure may be prepared as follows:

20 vol.-% of aqueous Spirulina extract per 1 liter of final solution (i.e., 200 ml) are mixed with 1000 mg sucrose (disaccharide of glucose and fructose, C₁₂H₂₂O₁₁) per 1 liter of final solution to form solution 1. Then the extracts given in Table 5 below are added in the following order and amount to solution 1 with subsequently oxygen (O₂) being added by bubbling gaseous oxygen through the solution at an oxygen pressure of 200 kPa over 1 hour resulting in solution 2. The concentrations of the corresponding extracts are listed in Table 6 below and may depend on the quality of the raw material for the extract.

**Table 5: Ingredients in Exemplary Composition A**

| No. | Extract | Amount [vol.-% per 1 liter final solution] |
|---|---|---|
| 1 | Agaricus subrufescens | 26 |
| 2 | Hypericum perforatum | 7 |
| 3 | Papaya | 20 |
| 4 | Pineapple | 20 |
| 5 | Sambucus flos | 7 |

**Table 6: Amount of plant and algae used for preparation of aqueous extract**

| Compound | Amount used for extraction (g/100 ml distilled water) | Compound condition |
|---|---|---|
| Cyanobacteria (Spirulina) | 10 | Dried |
| Agaricus Subrufescens | 20 | Dried |
| Pineapple | 100 | Fresh |
| Carica papaya | 100 | Fresh |
| Hypericum perforatum | 40 | Fresh |
| Sambucus flos | 40 | Fresh |

Solution 2 is then centrifuged at 500g for 15 min resulting in a centrifugate being solution 3. As centrifuge, any type of centrifuge may be taken, e.g. Heraeus Megafuge 40. After that, oxygen (O₂) is added by bubbling gaseous oxygen through solution 3 at an oxygen pressure of 200 kPa for 30 minutes resulting in the final solution. The final solution is termed Exemplary Composition A and used in the following Case Reports. Apart from said Exemplary Composition A, also Additives 1 to 3 as described above are used in the Case Reports. It has to be mentioned again that Additives 1 to 3 are not mandatory for the treatment of the above described indications.

### Production process of Exemplary Composition A with ingredients in dried form

20 g dried powders of Spirulina and 1000 mg sucrose (disaccharide of glucose and fructose, C₁₂H₂₂O₁₁) are poured with 200 ml distilled water at 50 °C. The suspension is allowed to brew for 7 minutes, followed by a filtration (filter pore size in the range of 0,1 to 1 µm, type of filter e.g. MILLEX®GP, manufacturer e.g. MILLIPORE, Bedford, MA), resulting in solution 1. Oxygen (O₂) is added by bubbling gaseous oxygen through solution 1 at an oxygen pressure of 200 kPa over 1 hour resulting in solution 2.

Aqueous extracts of agaricus subrufescens, hypericum perforatum, carica papaya, pineapple, sambucus flos may be made in accordance with the following procedure. Dried powder of the ingredient in the concentration listed in Table 6 is poured with distilled water at 50 °C. The suspension is allowed to brew for 7 minutes, followed by a filtration (filter pore size in the range of 0,1 to 1 µm, type of filter e.g. MILLEX®GP, manufacturer e.g. MILLIPORE, Bedford, MA), resulting in the corresponding aqueous extract. The aqueous extracts are mixed together in the order and amounts of Table 5, and subsequently oxygen (O₂) is added by bubbling gaseous oxygen through the solution at an oxygen pressure of 200 kPa over 1 hour resulting in the final solution being equivalent to Exemplary Composition A above.

Exemplary suppliers of the dried powders or ready to use extracts are given in Table 7:

**Table 7: suppliers of ingredients**

| **Ingredient** | **Supplier** | **Specification** |
|---|---|---|
| Spirulina | Earthrise (California,USA) | Spirulina Natural Powder "The One Powder" |
| Agaricus Blazei Murill | Hawlik Gesundheitsprodukte, Germany | Agaricus Extract |
| Hypericum perforatum | Dixa AG St. Gallen, Switzerland | European type |
| Carica Papaya | Spira Verde GmbH, Germany, or fresh papaya from Green Field Exports, India | Papaya fruit granulate with seeds |
| Pineapple | Flora Apotheke, Hannover, Germany, or fresh pineapple from Green Field Exports, India | Anavit F3 |
| Sambucus flos | Dixa AG St. Gallen, Switzerland | European type |

### Selected case Reports

### Case Report 1: Complete remission of a pT2 rectum adeno carzinoma

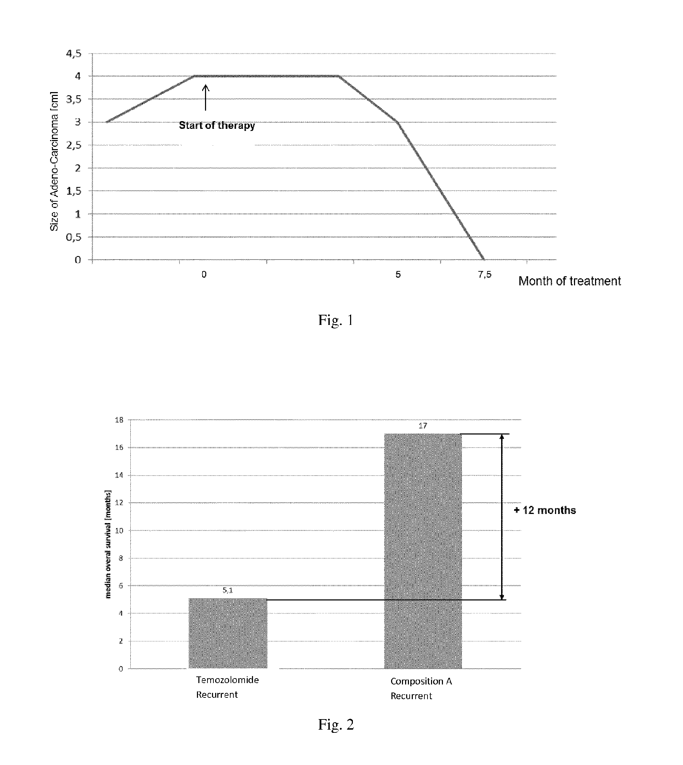

A female, 72 year old patient with pT2 adeno carcinoma in the rectum with no previous operation, chemotherapy or radiotherapy was treated with Exemplary Composition A with Additives 1 to 3 according to the dosage regimen given in Table 4 above. At the beginning of the treatment, the patient had an EORTC life quality of 1 and an EORTC physical condition of 1 (according to EORTC-QLQ C30 questionnaire (http://groups.eortc.be/qol/eortc-qlq-c30), level of 1 corresponding to "very bad", and 7 corresponding to "excellent"). Prior to the treatment, the tumor had a size of about 4 cm (see also Fig. 1 showing the size of the tumor (in cm) over the time (in months); the carcino-embryonic antigen (CEA) level was at 6.8 ng/ml.

During the first six months of treatment, the tumor was slightly regressing, and then strongly regressing during month 5 to 7,5 of the treatment. After 7,5 months there was only a residual wall thickening of the rectum left, but no sign of an adeno-carcinoma. The blood count and the liver as well as kidney values were without pathological findings during the whole course of the therapy; the EORTC life quality and the EORTC physical condition were both rated 6 at the end of the therapy after 8 months.

### Case Report 2: Complete remission of a glioblastoma multiforme IV

A male, 51 year old patient having a glioblastoma multiforme (WHO grade IV, left, occipital) had an operation, followed by the treatment with 5 different chemotherapy agents for more than two years (relapse during that time, thus three further operations) including thirty radiotherapy sessions. Prior to the treatment with Exemplary Composition A, the chemotherapy was stopped due to severe adverse effects. At that time, the patient could not walk but had to use a wheelchair, he could not speak fluently anymore and had ample cognitive deficits. He was then treated with Exemplary Composition A and with Additives 1 to 3 according to the dosage regimen given in Table 4 above. At the beginning of the treatment, the patient had an EORTC life quality of 2 and an EORTC physical condition of 2, and had no courage to face life. Due to the infiltrative growth of a glioblastoma there is in most cases a recurrent disease even after a complete resection of the glioblastoma. This leads to the highest mortality rate among all cancer diseases. The median survival rate of patients with a glioblastoma after a relapse surgery with a subsequent chemotherapy with temozolomide 5,1 months. The median overall survival of a newly diagnosed glioblastoma multiforme grade IV is 12,1 months with radiotherapy and 14,6 months with radiotherapy and chemotherapy (temozolomide) combined.

After four months of treatment, the patient has a stable condition, there are no indications for relapse, no metastasis. At the end of the treatment with Exemplary Composition A after ten months, a full remission of the tumor can be assessed; the patient has no relapse or metastasis. He can walk on his own and speak fluently. The blood count is without pathological findings, the gamma-GT level was increased due to an alcohol abuse. The EORTC life quality and the EORTC physical condition were both rated 5. At the date of this report, the overall survival time of the patient is 17 months, compared to a median overall survival time of patients with a recurrent glioblastoma being treated with Temozolomide of 5,1 months, as can also be seen in Fig. 2. But this patient is still alive with no relapse and at a very good health state.

### Case Report 3: Complete remission of an G3 invasive adeno carcinoma of the prostate

A male, 77 year old patient having an invasive adeno carcinoma of the prostate (Gleason Score 7 (3+4), G3 Mostofi/WHO) with florid periglandular prostatitis (histologically confirmed by 14 punch biopsies) with no previous operation, chemotherapy or radiotherapy was treated with Exemplary Composition A and with Additives 1 to 3 according to the dosage regimen given in Table 4 above. At the beginning of the treatment, the patient had an EORTC life quality of 2 and an EORTC physical condition of 2.

During eight months of treatment, the prostate-specific antigen (PSA) level was decreasing from 4.92 ng/ml at the beginning, 4.06 ng/ml after one month, 4.16 ng/ml after two months, to 3.63 ng/ml after three months and 3.77 ng/ml after four months. The therapy was continued for a total of eight months. The blood count and the liver values were without pathological findings during the whole course of the therapy. The EORTC life quality and the EORTC physical condition were both rated 6 at the end of the therapy. 4,5 months after the end of the therapy, an histological examination of the prostate was done, with the result that there was no confirmation of the clinically known prostate carcinoma.

### Case Report 4: Myoma Case 1

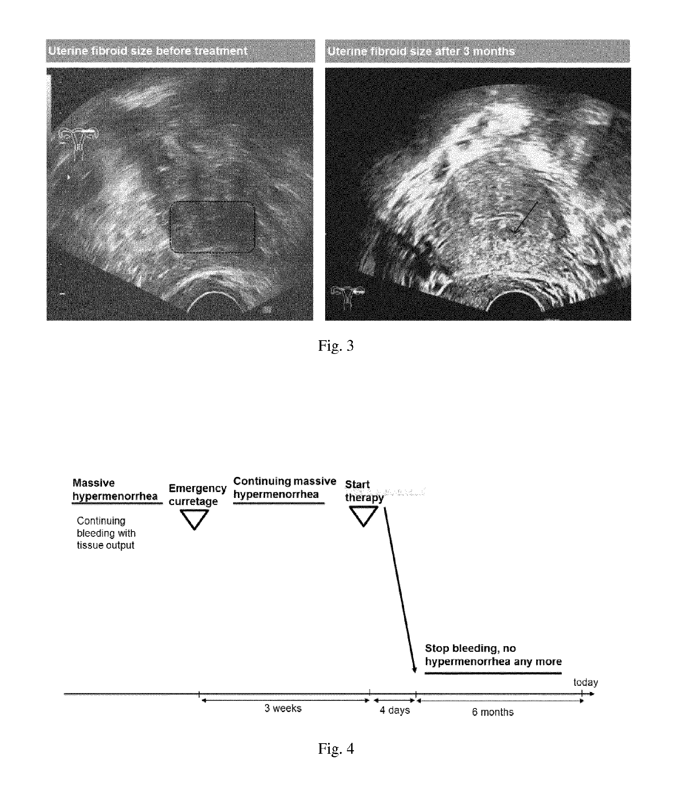

A female, 35 year old patient had 3 uterine myoma (up to 5 cm in size) and one submucosus myoma. The three uterine myoma were removed by operation. Within 3 months after the operation, 2 new myoma with sizes of 1,6 x 1,9 cm and 2 x 2,2 cm recurred. The patient had again heavy recurrent bleeding. The size of the uterine myoma is shown in Fig. 3, left hand side. The patient was then treated with Exemplary Composition A and with Additive 1 to 3 according to the dosage regimen given in Table 4 above. At the beginning of the treatment, the patient had an EORTC life quality of 4 and an EORTC physical condition of 3.

The myoma vanished almost completely within 3 months of the therapy (see Fig. 3, right hand side) and the heavy recurrent bleeding stopped totally within the same time frame. The blood count and the liver values were without pathological findings during the whole therapy course; the EORTC life quality was at 5 and the EORTC physical condition at 5 after the therapy.

### Case Report 5: Myoma Case 2

In a female, 53 year old patient, an intraductal mamma carcinoma was removed by operation and the patient was subsequently treated with tamoxifen. Then, the patient had a recurrent bleeding uterine myoma, on the right side (pT1c, pTis, G2), which is a typical symptom of an uterine myoma. It was proposed to have a hysterectomy due to a histological findings (focale, atypical, complex endometrium hyperplasia, grade III according to Dallenback-Hellweg, pre-stage of an unterine carcinoma). The patient was then treated with Exemplary Composition A and with Additives 1 to 3 according to the dosage regimen given in Table 4 above. At the beginning of the treatment, the patient had an EORTC life quality of 3 and an EORTC physical condition of 3.

Within the first few days of treatment, the bleeding stopped, and has not returned since. The therapy was continued for a total of six months. The blood count and the liver values were without pathological findings during the whole therapy course; the tumor marker M2-PK was at 1.1 U/ml (a level of above 4 U/ml is considered as an increased level); the EORTC life quality is at 5 and the EORTC physical condition at 4 at present. Within 9 months (present day) after the end of the therapy, there was no hypermenorrhea any more. In comparison thereto, with the currently available medication for myoma, a recurrent hypermenorrhea will usually occur within 3-5 months after the stop of the medication.

### Case Report 6: Myoma Case 3

A female, 50 year old patient had continuing bleeding with bloody tissue output due to an uterine myoma. The size of the uterine myoma was 4 cm. An emergency curettage of the uterine myoma was done, which caused a stoppage of the bleeding, but only for about 3 weeks. Then there was a massive hypermenorrhea. The uterine myoma was again 4 cm large despite the emergency curretage. Then, the female patient was treated with Exemplary Composition A and with the Additives 1 to 3 according to the dosage regimen given in Table 4 above. Within 4 days there was a complete stop of the massive hypermenorrhea, which did not recur since. The therapy lasted 4 months, though the massive hypermenorrhea stopped within 4 days. The blood count and the liver values were without pathological findings during the whole therapy course. The history of said patient is summarized in Fig. 4.

### Case Report 7: Complete remission of a meningioma

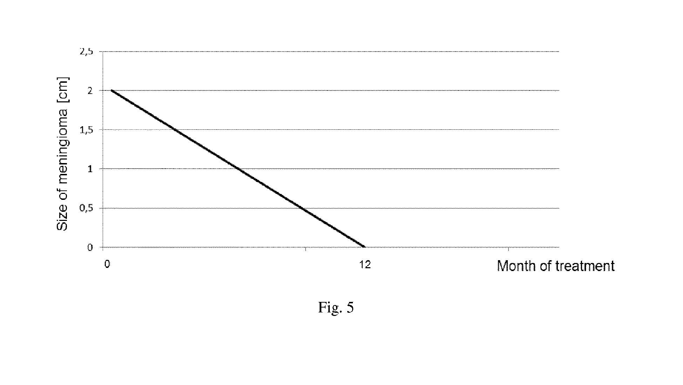

A female, 65 year old patient having a meningioma in the right cerebral hemisphere and multiple co-morbidity with no previous operation, chemotherapy or radiotherapy was treated with Exemplary Composition A with additives 1 to 3 according to the dosage regimen given in Table 4 above. At the beginning of the treatment, the patient had an EORTC life quality of 3 and an EORTC physical condition of 3. Prior to the treatment, the tumor had a size of about 2 cm (see also Fig. 5 showing the size of the tumor (in cm) over the time (in months). The clinical status of the patient at the beginning of the therapy was:
- Very acute headache for the for the last 6 years (start of meningioma)
- Strong disturbance of equilibrium sense
- Left leg and arm strongly asthenic and only limited functional
- 3 toes of left foot completely asthenic
- For the last 20 years renal insufficiency (for many years every quarter renal inflammation)
- Intake of drugs against pain

The treatment lasted for twelve months. Three months after the end of the treatment, the tumor was completely regressed (see Fig. 5), and the MRT showed no finding. The blood count and the liver values were without pathological findings during the whole course of therapy; the EORTC life quality and the EORTC physical condition were both rated 6.

The clinical status of the patient at the end of the therapy was:
- No headache any more
- Left arm: 100% improvement, fully functional
- 3 toes at left foot: 100% improvement, fully functional
- Left leg: 90% improvement, nearly fully functional
- Kidney: 80% improvement, no renal insufficiency any more
- Patient feels calm and relaxed, health status was never as good as today

As can be seen from the above given Case Reports 1 to 7, Exemplary Composition A is effective in treating tumors to the brain, colorectal cancer, prostatic cancer and benign tumors, such as myoma.

The present application furthermore also pertains to the following numbered embodiments:
1. A composition comprising the compounds *carica papaya* and *pineapple,* and at least one compound selected from the group consisting of *agaricus subrufescens; cyanobacteria; sambucus;* and *hypericum perforatum.*
2. Composition according to embodiment 1 comprising the compounds *carica papaya* and *pineapple,* and at least one compound selected from the group consisting of *agaricus subrufescens* and *cyanobacteria.*
3. Composition according to embodiment 1 comprising the compounds *carica papaya; pineapple; agaricus subrufescens;* and *cyanobacteria.*
4. Composition according to embodiment 2 or 3, additionally comprising at least one compound selected from the group consisting of *sambucus* and *hypericum perforatum.*
5. Composition according to any one of the preceding embodiments, wherein the *cyanobacteria* is *spirulina.*
6. Composition according to any one of the embodiments 1, or 3 to 5, wherein the *sambucus* is *sambucus flos.*
7. Composition according to any one of the preceding embodiments, comprising the compounds *carica papaya,* preferably green *carica papaya; pineapple; agaricus subrufescens;* and *cyanobacteria,* preferably *spirulina;* and at least one compound selected from the group consisting of *sambucus,* preferably *sambucus flos;* and *hypericum perforatum.*
8. Composition according to any one of the preceding embodiments, comprising the compounds *carica papaya,* preferably green *carica papaya; pineapple; agaricus subrufescens; cyanobacteria,* preferably *spirulina; sambucus,* preferably *sambucus flos;* and *hypericum perforatum.*
9. Composition according to any one of the preceding embodiments, wherein the compound is a plant or bacterium, preferably wherein the compound is an extract of said plant or bacterium, respectively, and more preferably an aqueous extract.
10. Composition according to any one of the preceding embodiments, wherein any of the compounds selected from the group consisting of *carica papaya,* preferably green *carica papaya; pineapple; agaricus subrufescens; cyanobacteria,* preferably *spirulina; sambucus,* preferably *sambucus flos;* and *hypericum perforatum,* is present as an extract, preferably an aqueous extract, in an amount of from 50 to 300 ml, preferably from 70 to 260 ml, based on one liter of composition.
11. Composition according to any one of the preceding embodiments, wherein any of the compounds selected from the group consisting of *carica papaya,* preferably green *carica papaya; pineapple; cyanobacteria,* preferably *spirulina; sambucus,* preferably *sambucus flos;* and *hypericum perforatum,* is present as an extract, preferably an aqueous extract.
12. Composition according to any one of the preceding embodiments, wherein the composition further comprises a sugar, preferably sucrose.
13. Composition according to any one of the preceding embodiments, wherein the composition further comprises oxygen (O₂), preferably containing oxygen (O₂) in an amount of more than 23 mg/l.
14. Composition according to any one of the preceding embodiments, wherein the composition further comprises calcium phosphate, potassium citrate, silicon dioxide, sodium chloride, magnesium citrate, lithium carbonate, zinc gluconate, hypericum perforatum, and cyanobacteria or agaricus subrufescens.
15. Composition according to embodiment 14, wherein the composition further comprises colloidal silver.
16. Composition according to any one of embodiments 14 to 15, wherein the composition further comprises vitamins and/or minerals.
17. Composition according to any one of the preceding embodiments, wherein the composition is an aqueous composition.
18. Use of a composition according to any one of the preceding embodiments 1 to 17 for the treatment or prevention of a benign and malign tumor indication.
19. Use according to embodiment 18, wherein the benign and malign tumor indication is selected from the group consisting of
   K1. Tumors to the brain;
   K2. Colorectal cancer with or without metastases;
   K3. Prostatic cancer with or without metastases; and
   K4. Benign tumor indications.
20. Use according to any one of embodiments 18 to 19, wherein the composition is administered orally, intravenously, subcutaneously, or topically, preferably orally or intravenously, and more preferably orally.
21. Use according to any one of embodiments 18 to 20, wherein the composition is administered once daily up to 10 times daily, preferably once daily up to five times daily, and more preferably three times daily.
22. Use according to any one of embodiments 18 to 21, wherein the treatment is applied for at least four weeks, preferably at least 12 weeks, and more preferably between 12 weeks and 48 weeks.
23. Use according to any one of embodiments 18 to 22, wherein the composition is co-administered together with at least one additive, which is not necessarily taken at the same time and/or mixed with the composition.
24. Use according to embodiment 23, wherein the at least one additive is selected from the group consisting of
   (a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof;
   (b) colloidal silver;
   (c) oxygen water, preferably containing oxygen (O₂) in an amount of more than 23 mg/l;
   (d) vitamin(s); and
   (e) mineral(s).
25. Use according to embodiment 23, wherein the composition is co-administered together with at least the additive
   (a) composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof.
26. Use according to embodiment 23, wherein the composition is co-administered together with at least the additive
   (a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof; and
   (b) colloidal silver.
27. Use according to embodiment 23, wherein the composition is co-administered together with at least the additives
   (a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof; and
   (c) oxygen water, preferably containing oxygen (O₂) in an amount of more than 23 mg/l.
28. Use according to embodiment 23, wherein the composition is co-administered together with at least the additives
   (a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof;
   (b) colloidal silver; and
   (c) oxygen water, preferably containing oxygen (O₂) in an amount of more than 23 mg/l.
29. Use according any one of embodiments 24 to 28, wherein the composition Q4 comprises a mixture of calcium phosphate, potassium citrate, silicon dioxide, sodium chloride, magnesium citrate, lithium carbonate, zinc gluconate, hypericum perforatum, and cyanobacteria or agaricus subrufescens.
30. Composition according to any one of embodiments 1 to 17 for use in the treatment or prevention of a benign and malign tumor indication.
31. Method for the treatment or prevention of a benign and malign tumor indication in an animal, comprising administering to an animal in need thereof, an effective amount of a composition of any one of embodiments 1 to 17.
32. Use of a composition according to any one of embodiments 1 to 17 for the preparation of a medicament.
33. Use of a composition according to any one of embodiments 1 to 17 for the preparation of a medicament for the treatment or prevention of a benign and malign tumor indication.
34. Method for the preparation of a composition according to any one of embodiments 1 to 17, wherein the composition is an aqueous composition, comprising the steps of
   (a) Providing a ready to use aqueous extract of *carica papaya* and *pineapple,* and admixing at least an aqueous extract of a compound selected from the group consisting of *agaricus subrufescens; cyanobacteria; sambucus;* and *hypericum perforatum;*
   (b) adding sucrose to said mixture; and
   (c) optionally enriching said mixture with oxygen.
35. Kit of part comprising
   - a composition according to any one of embodiments 1 to 17; and
   - an additive comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and a combination thereof; and/or
   - oxygen water, preferably containing oxygen (O₂) in an amount of more than 23 mg/l; and/or
   - colloidal silver; and/or
   - vitamin(s); and/or
   - mineral(s); and
   - optionally instructions for a dosage regimen.

## Claims

1. A composition comprising the compounds *carica papaya* and *pineapple,* and at least one compound selected from the group consisting of *agaricus subrufescens; cyanobacteria; sambucus;* and *hypericum perforatum.*

2. Composition according to claim 1 comprising the compounds *carica papaya,* preferably green *carica papaya; pineapple; agaricus subrufescens;* and *cyanobacteria,* preferably *spirulina;* and at least one compound selected from the group consisting of *sambucus,* preferably *sambucus flos;* and *hypericum perforatum.*

3. Composition according to any one of the preceding claims, wherein the compound is a plant or bacterium, preferably wherein the compound is an extract of said plant or bacterium, respectively, and more preferably an aqueous extract.

4. Composition according to any one of the preceding claims, wherein the composition further comprises a sugar, preferably sucrose, and/or oxygen (O₂), preferably containing oxygen (O₂) in an amount of more than 23 mg/l.

5. Composition according to any one of the preceding claims, wherein the composition further comprises calcium phosphate, potassium citrate, silicon dioxide, sodium chloride, magnesium citrate, lithium carbonate, zinc gluconate, hypericum perforatum, and cyanobacteria or agaricus subrufescens.

6. Composition according to claim 5, wherein the composition further comprises colloidal silver.

7. Composition according to any one of claims 5 to 6, wherein the composition further comprises vitamins and/or minerals.

8. Use of a composition according to any one of the preceding claims 1 to 7 for the treatment or prevention of a benign and malign tumor indication.

9. Use according to claim 8, wherein the benign and malign tumor indication is selected from the group consisting of
K1. Tumors to the brain;
K2. Colorectal cancer with or without metastases;
K3. Prostatic cancer with or without metastases; and
K4. Benign tumor indications.

10. Use according to any one of claims 8 to 9, wherein the composition is administered orally, intravenously, subcutaneously, or topically, preferably orally or intravenously, and more preferably orally.

11. Use according to any one of claims 8 to 10, wherein the composition is co-administered together with at least one additive, which is not necessarily taken at the same time and/or mixed with the composition.

12. Use according to claim 11, wherein the at least one additive is selected from the group consisting of
(a) a composition Q4 comprising at least one compound selected from the group consisting of calcium phosphate, potassium citrate, silicon dioxide, sodium chloride, magnesium citrate, lithium carbonate, zinc gluconate, hypericum perforatum, cyanobacteria and agaricus subrufescens, and combinations thereof;
(b) colloidal silver;
(c) oxygen water, preferably containing oxygen (O₂) in an amount of more than 23 mg/l;
(d) vitamin(s); and
(e) mineral(s).

13. Composition according to any one of claims 1 to 7 for use in the treatment or prevention of a benign and malign tumor indication.

14. Use of a composition according to any one of claims 1 to 7 for the preparation of a medicament.

15. Method for the preparation of a composition according to any one of claims 1 to 7, wherein the composition is an aqueous composition, comprising the steps of
(a) Providing a ready to use aqueous extract of *carica papaya* and *pineapple,* and admixing at least an aqueous extract of a compound selected from the group consisting of *agaricus subrufescens; cyanobacteria; sambucus;* and *hypericum perforatum;*
(b) adding sucrose to said mixture; and
(c) optionally enriching said mixture with oxygen.
